(19) Europäisches Patentamt
European Patent Office
Office européen des brevets

(11) Numéro de publication: **0 194 905**
**A1**

# (12) DEMANDE DE BREVET EUROPEEN

(21) Numéro de dépôt: **86400270.4**

(22) Date de dépôt: **10.02.86**

(51) Int. Cl.⁴: **C12N 9/12**, C12P 19/30, C07B 59/00, //(C12N9/12,C12R1:865)

(30) Priorité: **12.02.85 FR 8501980**

(43) Date de publication de la demande: **17.09.86 Bulletin 86/38**

(84) Etats contractants désignés: **AT BE CH DE FR GB IT LI LU NL SE**

(71) Demandeur: **Etablissement Public dit: CENTRE NATIONAL DE LA RECHERCHE SCIENTIFIQUE (CNRS)**
**15, Quai Anatole France**
**F-75700 Paris(FR)**

(72) Inventeur: **Blanquet, Sylvain**
**25, Boulevard Arago**
**F-75013 Paris(FR)**
Inventeur: **Guranowski, Andrzej**
**Osiedle B. Chrobrego I6m. 47**
**61683 Poznan(PL)**

(74) Mandataire: **Gillard, Marie-Louise et al**
**Cabinet Beau de Loménie 55, Rue d'Amsterdam**
**F-75008 Paris(FR)**

(54) **5',5'''-p1,p4-diadénosine-tétraphosphate alpha,b ta-phosphorylase et son utilisation pour la préparation de nucléotides isotopiquement marqués.**

(57) L'invention concerne une nouvelle enzyme la 5',5'''-P¹,P⁴-diadénosine-tétraphosphate α,β-phosphorylase extraite de levures, notamment de Sccharomyces cerevisiae. Cette enzyme permet d'obtention de nucléotides isotopiquement marqués à partir de substrats apprcpriés.

EP 0 194 905 A1

5,',5"'-P',P[4]-diadénosine-tétraphosphate α,β-phosphorylase et son utilisation pour la préparation de nucléotides isotopiquement marqués.

La présente invention concerne une nouvelle enzyme obtenue à partir d'extraits de levures, par exemple de Saccharomyces cerevisiae. Elle a également pour objet l'utilisation de cette enzyme, la diadénosine-tétraphosphate α,β-phosphorylase, pour l'obtention de nucléotides isotopiquement marqués. On a en effet trouvé que cette nouvelle enzyme dégrade le 5',5"'-P',P[4]-diadénosine-tétraphosphate, dénommé ci-après "$Ap_4A$", en le phosphorylant.

L'$Ap_4A$ est le produit d'une réaction secondaire de l'activation des aminoacides catalysée par la lysyl-tARN-synthétase d'Escherichia coli. On a montré que cette synthétase est également capable de produire une nouvelle famille de composés de formules générales $Ap_3N$ ou $Ap_4N$ dans lesquelles N est un nucléoside purique ou pyrimidique.

Plus récemment, il a été trouvé que plusieurs aminoacyl-tARN-synthétases de différents organismes, tels que les bactéries, les champignons, les mammifères et les plantes supérieures, sont capables de synthétiser efficacement in vitro l'$Ap_4A$ et l'$Ap_3A$ (5',5"'-P',P[3]-diadénosine-triphosphate).

On suppose que ces composés sont produits in vivopar ces synthétases. La présence d'$Ap_4A$ a été démontrée dans un certain nombre de systèmes vivants, tels que les tissus de mammifères, les bactéries, les protozoaires, les invertébrés et les organismes Physarum polycephalum et Saccharomyces cerevisiae. L'$Ap_3A$ a été récemment identifié à la fois dans les cellules eucaryotes et les cellules procaryotes.

A côté des composés cités ci-dessus, il existe dans la nature une autre famille de dinucléosides oligophosphates, il s'agit des composés guanylés $Gp_4G$, $Gp_3G$, $Gp_2G$ et $Ap_3G$ qui ont été identifiés chez certains crustacés. Dans ces formules, G désigne la guanosine. Les produits $Gp_4G$ et $Gp_3G$, qui sont respectivement le diguanosine tétraphosphate et le diguanosine triphosphate sont synthétisés par la guanylyltransférase chez plusieurs organismes. Récemment, il a été montré qu'une enzyme purifiée obtenue à partir de levure produit in vitro des dinucléosidestri-et tétra-phosphates de type $Gp_3N$ et $Gp_4N$, dans lesquels N désigne l'adénosine, la guanosine, la cytidine ou l'uridine.

Le rôle biologique des dinucléosides oligophosphates n'est pas très bien connu. Cependant, on sait que les dinucléosides oligophosphates $Gp_4G$ et $Ap_4G$ peuvent réguler le métabolisme des purines. On a trouvé par ailleurs que le $Gp_4G$ a un effet positif spécifique sur la GMP-réductase obtenue à partir d'Artemia et d'érythrocytes humains et que l'$Ap_4A$ double l'activité de l'AMP-désaminase obtenue à partir de muscles de rat. Enfin, on pense que l'$Ap_4A$ intervient dans la régulation de la réplication de l'ADN. Son taux dans les cellules varie en fonction de l'activité proliférante des tissus ou avec le cycle des cellules et, en interagissant avec l'ADN-polymérase α, il amorcerait la synthèse de l'ADN.

L'hypothèse que les dinucléosides oligophosphates jouent un rôle important dans le métabolisme cellulaire est renforcée par le fait qu'en plus des enzymes qui peuvent synthétiser ces produits il existe des activités enzymatiques qui les catabolisent. Dans les extraits de cellules eucaryotes, l'$Ap_4A$ et ses analogues sont attaqués par une phosphodiestérase qui libère le nucléoside 5'-monophosphate. De plus, chez certains organismes, on a trouvé deux enzymes distinctes hautement spécifiques qui hydrolysent les dinucléosides oligophosphates. L'une d'entre elles, dénommée $Ap_4A$-hydrolase ou dinucléoside tétraphosphatase (EC 3.6.1.17), trouvée dans de nombreuses espèces, coupe préférentiellement les dinucléosides tétraphosphates et un nucléoside 5'-triphosphate est toujours l'un des produits de la réaction enzymatique. L'autre enzyme, dénommée $Ap_3A$-hydrolase (EC 3.6.1.29), trouvée dans les tissus de rat et les graines de lupin, utilise de préférence l'$Ap_3A$ comme substrat, mais hydrolyse aussi d'autres dinucléosides oligophosphates et un nucléoside 5'-diphosphate est toujours un produit de la réaction.

Chez les bactéries et quelques organismes eucaryotes inférieurs, tels que Physarum polycephalum et Euglena gracilis, on a identifié un autre type d'$Ap_4A$-hydrolase. Cette hydrolase coupe la molécule d'$Ap_4A$ de façon symétrique pour donner deux molécules d'ADP [L.D. BARNES et C.A CULVER Biochemistry 21, 61236128 (1982); GURANOWSKI et al. J. Biol. Chem. 258, 14784-14789 (1983)].

On a maintenant trouvé que l'$Ap_4A$ et certains autres dinucléosides oligophosphates peuvent être dégradés phosphorolytiquement par action d'une nouvelle enzyme provenant d'extraits de levures, notamment de la levure Saccharomyces cerevisiae .

L'enzyme selon l'invention, dénommée 5',5"'-P',P[4]-diadénosine tétraphosphate α,β-phosphorylase ou $Ap_4A$-α,β-phosphorylase est obtenue à partir d'extraits bruts de levure en mettant en oeuvre des moyens classiques de séparation et de purification. Par exemple, elle peut être obtenue par fractionnement, à l'aide de sulfate d'ammonium, d'un extrait brut de Saccharomyces cerevisiae et purification par filtrations et chromatographies successives sur différents gels.

Cette enzyme, qui sera décrite plus en détail ci-après, catalyse la coupure phosphorolytique de l'une des liaisons anhydrides entre les résidus phosphates α et β de l'$Ap_4A$ ou d'autres dinucléosides oligophosphates. Le phosphate substrat du milieu se retrouve toujours incorporé dans le nucléoside 5'-diphosphate correspondant produit.

L'enzyme selon l'invention permet notamment l'incorporation d'isotopes, radioactifs ou non, de l'ion $PO_4^-$ en position β du 5'-ADP, du 5'-GDP ou d'un 5'-NDP formés au cours de la dégradation enzymatique selon les réactions suivantes :

$$ApppA + p^* \longrightarrow Appp + p^*pA$$

$$GppppG + p^* \longrightarrow Gppp + p^*pG$$

$$NppppN + p^* \longrightarrow Nppp + p^*pN$$

où p* désigne le phosphate à incorporer.

L'$Ap_4A$-α,β-phosphorylase catalyse également une réaction d'échange entre le β-phosphate d'un nucléoside diphosphate (NDP) et de l'orthophosphate marqué selon le schéma réactionnel :

Npp + p* ⟶ Npp* + p

Ainsi, la présente invention concerne également l'utilisation de la diadénosine tétraphosphate α,β-phosphorylase pour la préparation de nucléotides isotopiquement marqués, soit par dégradation phosphorolytique d'$Ap_4A$ ou d'autres dinucléosides oligophosphates contenant au moins quatre résidus phosphates liant les nucléotides par leur carbone 5', soit par échange entre le β-phosphate d'un nucléoside diphosphate et de l'orthophosphate marqué.

Elle concerne en particulier l'utilisation de cette enzyme pour l'incorporation de $^{32}P$ et celle des isotopes de l'oxygène $^{18}O$ et $^{17}O$.

L'utilisation de kinases connues permet ensuite les conversions ci-après :

P*pA (ou G) + p*pA (ou G) —kinase⟶ A (ou G) pp*p* + A (ou G) p

N + App*p* ⟶ Np* + App*

Np + App*p* ⟶ Npp* + App*

L'enzyme selon l'invention, couplée à ces kinases, permet donc l'obtention de différents mono-, di-et tri-nucléotides isotopiquement marqués.

L'invention concerne également les nucléotides isotopiquement marqués ainsi obtenus.

On va maintenant décrire en dètail l'obtention de l' $Ap_4A$-α,β-phosphorylase à partir d'un extrait brut de Saccharomyces cerevisiae sans pour autant limiter l'invention.

L'extrait de Saccharomyces cerevisiae a été préparé selon le mode opératoire ci-après.

700 g de poids humide de cellules fraîches de levure de boulanger Saccharomyces cerevisiae ont été mis en suspension dans 700 ml de phosphate de potassium 100 mM (pH 7,8) contenant 1 mM de dithioérythritol et 1 mM de fluorure de phénylméthylsulfonyle. Les cellules ont ensuite été rompues par trois passages de la suspension ainsi obtenue dans une presse Menton-Gaulin réfrigérée (600 kg/cm²).

L'extrait résultant a été dilué dans du glycérol 10% V/V final et centrifugé pendant 60 min à 20 000 g. Le surnageant obtenu, qui constitue l'extrait brut, a été soumis à un fractionnement avec du sulfate d'ammonium (50-70 % de saturation).

Le précipité obtenu a été dissous dans un tampon phosphate contenant 20 mM de phosphate de potassium - (pH 6,8),5 mM de 2-mercaptoéthanol et 10 % de glycérol, dénommé ci-après tampon B, et dialysé contre ce tampon B. Après centrifugation, le surnageant (380 ml) a été mélangé avec du DEAE-Séphacel (600 ml de gel compact) et la suspension obtenue a été versée dans une colonne.

Après tassement du gel, la colonne (5 x 30 cm) a été lavée avec 3 l du tampon B et ensuite un gradient linéaire 0-0,5 M de KCl dans le même tampon B a été appliqué - (au total 5,8 l).

L'activité phosphorylase, déterminée selon le mode opératoire défini ci-après, contenue dans les fractions 200-300 mM KCl, a été concentrée par précipitation à l'aide de sulfate d'ammonium (70 % de saturation). Le précipité a été récolté par centrifugation, dissous dans du tampon contenant 50 mM de phosphate de potassium (pH 6,8), 5 mM de 2-mercaptoéthanol et 10 % de glycérol, dénommé ci-après "tampon C" et chromatographié sur une colonne de "Sephadex G-100" (4,4 x 72 cm). Les fractions actives, qui sont apparues à Ve/Vo = 1,75 (Ve = Volume d'élution et Vo = Volume mort) ont été rassemblées et l'enzyme a été précipitée par du sulfate d'ammonium. Après resolubilisation et dialyse contre du tampon contenant 10 mM de phosphate de potassium (pH 6,8), 5 mM de 2-mercaptoéthanol et 10 % de glycérol, dénommé ci-après "tampon A", l'$Ap_4A$-phosphorylase a été passée sur une colonne de Bio-Gel HTP de Bio-RAD (hydroxyapatite; 1 x 12 cm). Après lavage avec 30 ml du tampon A, on a appliqué un gradient linéaire : 10-150 mM de phosphate de potassium - (pH 6,8) contenant 5 mM de 2-mercaptoéthanol et 10 % de glycérol (au total un volume de 120 ml). L'enzyme a éte éluée entre 50 et 80 mM de phosphate de potassium.

Elle a été concentrée avec du sulfate d'ammonium, dissoute dans un petit volume (1 ml) de tampon C et chromatographiée sur une colonne de Sephadex G-75 superfine (1,14 x 114 cm) équilibrée avec le tampon C.

On a recueilli des fractions de 1 ml. L'activité $Ap_4A$-phosphorylase était dans les fractions 46 à 48, correspondant au plus grand pic de densité optique à 280 nm.

L'activité de l'$Ap_4A$-phosphorylase a été déterminée à 37°C. Le mélange d'incubation standard contenait, dans un volume final de 25 ou 50 µl, du tampon 50 mM acide 4-(2-hydroxyéthyl)-1-pipérazine éthane sulfonique (Hepes)/KOH (pH 8,2), 1 mM 2-mercaptoéthanol, 2 % de glycérol, 500 µM $MnCl_2$, 10 mM $K_2HPO_4$, 500 µM de [$^3H$]$Ap_4A$ (1 pmol = 20 cpm), de la sérum-albumine bovine (0,4 mg/ml) et une solution d'enzyme diluée de façon appropriée en présence de 2 mg/ml de sérum-albumine bovine. Dans certains cas, l'activité a été suivie à l'aide de [$^{32}P$]$K_2HPO_4$.

Pour mesurer les vitesses initiales, de phosphorolyse, on a déposé des prélèvements de 2 µl sur des couches minces de polyéthylèneimine-cellulose contenant un indicateur fluorescent (fournies par MERCK) après 2, 4, 8 et 16 min de réaction.

Des standards d'$Ap_4A$ et d'ADP ont été ajoutés et les chromatogrammes ont été développés tout d'abord pendant 15 min dans le méthanol à 75 %, puis pendant 1 h dans du chlorure de lithium 0,85 M. Les valeurs $R_f$ des composés standards étaient respectivement de 0,15 ($Ap_4A$) et 0,25 (ADP), la valeur $R_f$ du phosphate était de 0,55. Les taches d'ADP visualisées sous lumière ultraviolette ont été découpées et immergées dans 5 ml de mélange de scintillation et la radioactivité a été mesurée. Au cours des différentes étapes de purification, l'enzyme a été testée en l'absence de radioactivité; les fractions actives ont été alors directement identifiées sur les chromatogrammes sous lumière ultraviolette.

Les protéines ont été repérées par mesure de l'absorption à 280 nm. La concentration en protéines a été déterminée par la méthode turbidimétrique au tannin selon la méthode de MEJBAUM-KATZENELENBOGEN, W - (1955) Acta Biochim. Polon. 2, 279-290.

Les caractéristiques des différentes fractions obtenues au cours du procédé d'obtention de l'enzyme $Ap_4A$-phosphorylase sont indiquées dans le tableau I ci-après.

Dans l'extrait brut et dans la fraction contenant l'$Ap_4A$-phosphorylase après précipitation au sulfate d'ammonium, on a observé une autre activité qui provoquait la dégradation de l'$Ap_4A$ en présence ou en absence de phosphate. Il s'agit probablement d'une phosphodiestérase non spécifique. En conséquence, le taux de purification indiqué dans le tableau I est sous-estimé. Par ailleurs, l'augmentation de l'activité totale observée après la précipitation au sulfate d'ammonium suggère la présence d'inhibiteurs de l'$Ap_4A$-phosphorylase dans l'extrait brut.

Après l'étape de purification sur gel de DEAE-Sephacel, l'$Ap_4A$-phosphorylase était dépourvue d'activité phosphodiestérase. Cette étape élimine aussi des activités du type ATPase et apyrase qui ne sont pas adsorbées sur le gel et l'activité adénylate kinase qui est retardée sur la colonne et éliminée par l'étape de lavage.

La séparation sur colonne de Sephadex G-100 permet d'éliminer les traces de quelques autres ATPases non identifiées.

Après la chromatographie sur hydroxyapatite, l'enzyme présente une pureté d'environ 40 %.

La chromatographie de la fraction ainsi obtenue sur des colonnes de phosphocellulose, de DEAE-Sephacel ou d'hydroxyapatite à des pH plus élevés que ceux utilisés précédemment, à savoir 7,8, ne permet pas d'éliminer les protéines contaminantes.

Par contre la filtration sur gel de Sephadex G-75 superfine est très efficace et la pureté de l'enzyme obtenue après cette dernière étape de purification est supérieure à 95 %.

Le poids moléculaire de la phosphorylase isolée a été estimé par filtration sur colonne de gel de Sephadex G-75 superfine (1 x 53 cm) selon la méthode de ANDREWS P. - (1964) Biochem. J. 91222-233. Cette détermination a été faite par comparaison avec les volumes d'élution des protéines standards suivantes :

| | |
|---|---|
| - cytochrome c de coeur de cheval | 14 500 |
| - anhydrase carbonique | 30 000 |
| - ovalbumine | 45 000 |
| - sérum-albumine bovine | 68 000 |

On a trouvé par cette méthode que le poids moléculaire de l'enzyme isolée était de 40 000.

Par électrophorèse sur gel de SDS-polyacrylamide selon la méthode de LAEMMLI U.K. (1970) Nature - (Londres) 227, 680-685, on a trouvé que l'$Ap_4A$-phosphorylase dénaturée présentait une seule bande de poids moléculaire 40 000. Ces résultats indiquent que l'$Ap_4A$ phosphorylase est constituée d'une seule chaîne polypeptique.

La figure 1 annexée est la photographie du gel d'électrophorèse sur laquelle les bandes A, $A_1$, $A_2$ et $A_3$ correspondent aux fractions d'$Ap_4A$-phosphorylase obtenues sur gel d'hydroxyapatite, respectivement les fractions combinées (A), la fraction de tête ($A_1$), la fraction médiane ($A_2$) et la fraction de queue ($A_3$) du pic de l'activité enzymatique. La bande B correspond aux protéines standards qui sont respectivement de haut en bas :

| | |
|---|---|
| méthionyl-tARN-synthétase de E.coli | (76 000) |
| méthionyl-tARN-synthétase de E. coli après digestion par la trypsine | (64 000) |
| ovalbumine | (45 000) |
| anhydrase carbonique | (30 000) |

la bande C correspond à l'$Ap_4A$-phosphorylase obtenue après filtration sur Sephadex G-75 superfine.

La dégradation enzymatique de l'$Ap_4A$ par l'enzyme selon l'invention, c'est-à-dire l'$Ap_4A$-phosphorylase, a été caractérisée par identification des produits de ladite réaction de dégradation par co-migration avec des composés standards authentiques sur des couches minces de polyéthylèneimine-cellulose. Cette identification a été confirmée sur une colonne de Lichrosorb RP-18 (MERCK) en utilisant un système HPLC (chromatographie liquide haute performance) avec une élution isocratique par du phosphate de potassium 25 mM, pH 5,3 selon la méthode de BREVET et al. décrite dans J.Biol.Chem. (1982), 257, 14613-14615. La colonne de chromatographie a été utilisée avec une pompe modèle 112, un injecteur modèle 210 et un détecteur d'absorbance modèle 160 (matériel BECKMAN), en opérant à 254 nm avec une sensibilité de 0,002 unité d'absorbance pleine échelle.

En présence de phosphate, on a constaté que l'$Ap_4A$ est converti par l'$Ap_4A$-phosphorylase en ATP plus ADP.

Par contre, aucune conversion n'a été observée en l'absence de phosphate ou lorsque le phosphate était remplacé par 10 mM de pyrophosphate ou de sulfate, que ce soit en présence de 500 $\mu$M de $MnCl_2$ ou en présence de 5 mM de $MgCl_2$.

Cependant, en présence d'arséniate, l'$Ap_4A$ subit également une dégradation avec 10 mM d'arséniate, la vitesse initiale de dégradation de l'$Ap_4A$ est seulement inférieure de 20 % à celle mesurée en présence de 10 mM de phosphate.

On a constaté que l'arsénolyse de l'$Ap_4A$ catalysée par l'$Ap_4A$-phosphorylase fournissait de l'ATP et de l'AMP. La production d'AMP à la place d'ADP suggère que l'adényl-arséniate formé est immédiatement décomposé en AMP et arséniate. Le résultat de cette décomposition arsénolytique de l'$Ap_4A$ indique que l'arséniate attaque l'une des liaisons anhydrides entre les résidus phosphate $\alpha$ et $\beta$ de l'$Ap_4A$.

Pour confirmer le mode de coupure phosphorolytique de l'$Ap_4A$ catalysée par l'enzyme selon l'invention, l'$Ap_4A$ a été incubé en présence de [$^{32}$p] orthophosphate. Parmi les deux produits formés (ATP et ADP), seulement l'ADP était marqué. Ces résultats excluent le fait que la phosphorolyse puisse avoir lieu au niveau de la liaison $\beta,\beta$-phosphate de l'$Ap_4A$.

Parmi les composés testés, le diadénosine 5',5'''-P$^1$,p$^4$-tétraphosphate ($Ap_4A$) est le substrat préféré de l'enzyme selon l'invention.

On a recherché parmi les composés structurellement apparentés à l'$Ap_4A$ les substrats potentiels de l'enzyme selon l'invention. Les composés ci-après ont été testés :

- Adénosine diphosphate (ADP)

-Adénosine triphosphate (ATP)

-Adénosine tétraphosphate ($p_4A$)

-Acide polyadénylique (poly A)

-Adénosine monophosphate 3',5'-cyclique (3',5' c AMP)

-Ester p-nitrophénylique de thymidine 5'-monophosphate

-Diadénosine diphosphate ($Ap_2A$)

-Diadénosine triphosphate ($Ap_3A$)

-Diadénosine pentaphosphate ($Ap_5A$)

-Diguanosine tétraphosphate ($Gp_4G$)

Parmi ces composés, seulement l'$Ap_5A$ et le $Gp_4G$ se sont comportés comme substrats de l'$Ap_4A$-phosphorylase et les produits résultant de la dégradation étaient respectivement $p_4A$ et ADP d'une part et GTP et GDP d'autre part.

En présence de [$^{32}$p] orthophosphate, le phosphore marqué a été retrouvé seulement dans les nucléosides diphosphates.

Les vitesses de phosphorolyse de l'$Ap_5A$ et du $Gp_4G$, mesurés dans un milieu d'incubation standard contenant 500 $\mu$M de substrat, correspondaient respectivement à 50 et 40 % du taux observé pour l'$Ap_4A$.

Finalement, on a noté que l'arsénolyse de l'$Ap_5A$ et du $Gp_4G$ fournissait $p_4A$ + AMP d'une part et GTP + GMP d'autre part.

Les produits formés au cours de l'arsénolyse ou de la phosphorolyse de $Ap_5A$ et $Gp_4G$ confirment le mécanisme de réaction indiqué précédemment, c'est-à-dire l'attaque enzymatique de l'une des liaisons anhydrides entre les deux résidus phosphates $\alpha$ et $\beta$ du substrat et l'incorporation du phosphate dans le nucléoside 5'-di-phosphate.

Le fait que le $p_4A$ et l'$Ap_3A$ ne soient pas des substrats de l'enzyme selon l'invention indique clairement que le site catalytique de l'enzyme nécessite d'une part la présence de deux résidus nucléotides dans le substrat et d'autre part une chaîne d'au moins quatre résidus phosphate liant les nucléosides par leurs carbones 5'.

L'effet du pH sur les vitesses initiales de phosphorolyse d'$Ap_4A$ a été déterminé. Ces vitesses ont été mesurées à différents pH en présence de 500 $\mu$M de [$^3$H]$Ap_4A$; 500 $\mu$M de $MnCl_2$; 5 mM de $K_2HPO_4$ (pH 8,0); 2 % de glycérol et 1 mM de 2-mercaptoéthanol. La gamme de pH de 5,6 à 8,8 a été obtenue en utilisant les tampons ci-après :

50 mM Mes/KOH; 50 mM Hepes/KOH; 50 mM Tricine/KOH

On a trouvé que le pH optimum pour l'activité phosphorolytique de l'enzyme était de 8,0 ± 0,2. On a par ailleurs noté qu'à pH 6,5 et 9,0 l'activité enzymatique correspondait à la moitié de l'activité maximale.

L'intégrité des cystéines de l'enzyme est essentielle à l'activité. En présence d'$Ap_4A$ saturant, la vitesse de la réaction de phosphorolyse est de 36 $s^{-1}$. La constante de Michaelis Km de l'$Ap_4A$ est de 60 $\mu$M (37°C, 50 mM Hepes/KOH pH = 8,2, 500 $\mu$M $MnCl_2$, 10 mM $K_2HPO_4$, 1 mM 2-mercaptoéthanol et 2 % de glycérol). Le Km du phosphate est de 1 mM.

La phosphorolyse de l'$Ap_4A$ par l'$Ap_4A$-phosphorylase n'a lieu qu'en présence de certains ions métalliques divalents.

Les vitesses initiales de la phosphorolyse de l'$Ap_4A$ ont été mesurées dans un milieu contenant 50 mM Hepes/KOH (pH 8,2); 1 mM de 2-mercaptoéthanol; 2 % de glycérol et 0,4 mg/ml de sérum-albumine bovine, 5 mM $K_2HPO_4$; 500 $\mu$M [$^3$H] $Ap_4A$ et différentes concentrations de chlorures de manganèse, de magnésium, de calcium, de cobalt ou de cadmium.

Les résultats obtenus sont indiqués sur le graphe de la figure 2 annexée sur lequel on a porté en ordonnées les vitesses initiales de phosphorolyse de l'$Ap_4A$ exprimées en pourcentages de la vitesse observée avec 500 $\mu$M $MnCl_2$ - (36 $s^{-1}$) et en abscisses la concentration en ions métalliques exprimée en mM.

Les différentes courbes correspondent respectivement aux chlorures ci-après : courbe A (O — O) = $MnCl_2$

courbe B (● — ●) = $MgCl_2$

courbe C (■ — ■) = $CaCl_2$

courbe D (□ — □) = $CdCl_2$

courbe E (X — X) = $CoCl_2$

Les résultats obtenus montrent que les ions $Mn^{2+}$ et $Mg^{2+}$ sont les plus actifs.

L'enzyme selon l'invention peut également être utilisée pour préparer des nucléotides isotopiquement marqués à partir de dinucléosides diphosphates par réaction d'échange avec le phosphate.

Les dinucléosides diphosphates qui peuvent être utilisés sont aussi bien les ribonucléosides diphosphates puriques ou pyrimidiques que les analogues de ces nucléotides modifiés, soit au niveau de l'aglycone ou du sucre, soit au niveau de la liaison anhydride en position $\beta$.

On a estimé les constantes de Michaelis Km et les vitesses d'échange pour différents NDP au pH optimum, lequel varie en fonction des substrats. Les résultats obtenus sont rassemblés dans le tableau II ci-après ainsi que les conditions expérimentales.

On notera que, contrairement à la réaction de dégradation phosphorolytique, la réaction d'échange NDP-P* ne requiert pas la présence d'ions métalliques divalents. En fait ces ions inhibent la réaction d'échange.

L'enzyme selon l'invention présente une spécificité pour l'adénosine 5'-phosphosulfate (AMPS) plus élevée que pour les autres NDP testés. Le rapport V/km est environ 5 fois plus élevé pour l'AMPS que pour l'ADP. En présence d'orthophosphate l'AMPS est irréversiblement transformé en ADP. Ainsi l'$Ap_4A$-phosphorylase de l'invention présente une propriété qui est analogue à celle de l'ADP-sulfurylase de levure (EC 2.7.7.5.) [GRUNBERG-MANAGO et al. Biochem. Biophys. Acta (1966) 123, 1-16; NICHOLLS R.G. Biochem. J. (1977) 165, 149-155].

Tableau I

Purification de la diadénosine tétraphosphate α,β-phosphorylase de Saccharomyces cerevisiae à partir de 700 g de poids humide de cellules de levure

| Fraction | Protéine | Activité spécifique[a] | Purification | Rendement |
|---|---|---|---|---|
| | mg | µmol $Ap_4A$/min/mg | (taux de) | % |
| extrait brut surnageant 20 000 x g | 25 200 | 0,033[b] | 1 | 100 |
| précipité au sulfate d'ammonium dialysé (50-70 %) | 14 250 | 0,115[b] | 3,5 | 197 |
| DEAE-Sephacel | 1 046 | 0,464 | 14 | 58 |
| Sephadex G-100 | 38 | 5,93 | 180 | 27 |
| Hydroxyapatite | 7,8 | 18,1 | 548 | 17 |
| Sephadex G-75 superfine | 2,4 | 55 | 1 666 | 16 |

a. l'activité a été mesurée dans un milieu contenant un tampon 50 mM Hepes/KOH (pH 8,2), 500 µm $MnCl_2$, 500 µM [$^3$H]$Ap_4A$, 10 mM $K_2HPO_4$, 2 % glycérol et 1 mM 2-mercaptoéthanol. L'incubation a été effectuée à 37°C.

b. Etant donné les activités dégradant l'ADP dans l'extrait brut et dans le précipité au sulfate d'ammonium dialysé, la disparition de l'$Ap_4A$ a été mesurée plutôt que l'apparition d'ADP.

Tableau II

Caractéristiques cinétiques de l'ADP et de ses analogues dans la réaction d'échange NDP-P* catalysée par l'$Ap_4A$-phosphorylase

| Substrat | pH optimum (tampon) | Vitesse à 2 mM de NDP ($s^{-1}$) (a) | Vitesse à saturation de NDP ($s^{-1}$) (b) | Km (mM) |
|---|---|---|---|---|
| ADP | 7,0 (Hepes) | 36 | 96 | 0,74 |
| analogues de l'ADP modifiés sur l'aglycone | | | | |
| GDP | 8,0 (Hepes) | 27 | 64 | 0,7 |
| IDP | 6,75 (Hepes) | 16 | | |
| XDP | 6,75 (Hepes) | 0,1 | | |
| CDP | 7,0 (Hepes) | 14 | 61 | 2,8 |
| UDP | 6,5 (Mes) | 7 | 27 | 3,7 |
| analogues de l'ADP modifiés sur le sucre | | | | |
| d-ADP | 6,75 (Hepes) | 16 | | |
| 2'-O-méthyl ADP | 7,0 (Hepes) | < 0,01 | | |
| analogues de l'ADP modifiés au niveau de la liaison anhydride en position β | | | | |
| ADPβS | 6,5 (Mes) | 2 | | |
| AMPS | 8,0 (Hepes) | 58 | 313 | 0,5 |
| $AMPPCH_2P$ | 7,0 (Hepes) | < 0,005 | | |

a) Le mélange d'incubation contenait 50 mM de tampon au pH optimum indiqué, 0,1 mM d'acide éthylène diamine tétraacétique (EDTA), 2,5 mM [$^{32}$P]$K_2HPO_4$, 2 mM NDP et la quantité d'enzyme $Ap_4A$-phosphorylase limitant la vitesse d'échange. L'incubation a eu lieu à 37°C.

b. Le mélange d'incubation contenait 100 mM de tampon au pH optimum indiqué, 0,1 mM d'EDTA, 10 mM [$^{32}$P]$K_2HPO_4$; NDP saturant et la quantité d'enzyme limitant la vitesse d'échange. L'incubation a eu lieu à 37°C.

## Revendications

1. 5',5'''-$P^1$,$P^4$-diadénosine-tétraphosphate $\alpha,\beta$-phosphorylase isolée à partir d'extraits bruts de levures.

2. 5',5'''-$P^1$,$P^4$-diadénosine-tétraphosphate $\alpha,\beta$-phosphorylase de Saccharomyces cerevisiae ayant un poids moléculaire de 40 000 obtenue par fractionnement au sulfate d'ammonium d'un extrait brut de Saccharomyces cerevisiae et chromatographies successives sur DEAE-Sephacel, Sephadex G-100, hydroxyapatite et Sephadex G-75 superfine.

3. Utilisation de 5',5'''-$P^1$,$P^4$-diadénosine-tétraphosphate $\alpha,\beta$-phosphorylase isolée à partir d'extraits bruts de levures pour la préparation de nucléotides isotopiquement marqués.

4. Utilisation selon la revendication 3, caractérisée en ce que la 5',5'''-$P^1$,$P^4$-diadénosine-tétraphosphate $\alpha,\beta$-phosphorylase est obtenue à partir d'extraits bruts de Saccharomyces cerevisiae et en ce qu'elle a un poids moléculaire de 40 000.

5. Utilisation selon l'une des revendications 3 ou 4, caractérisée en ce que les nucléosides diphosphates isotopiquement marqués sont obtenus par dégradation phosphorolytique de substrats constitués de nucléosides oligophosphates contenant deux nucléosides reliés entre eux par leurs carbones 5' par l'intermédiaire d'une chaîne comprenant au moins 4 résidus phosphates.

6. Utilisation selon la revendication 5, caractérisée en ce qu'on opère à pH 8 environ.

7. Utilisation selon l'une des revendications 5 ou 6, caractérisée en ce que l'on opère en présence d'ions métalliques divalents.

8. Utilisation selon la revendication 7, caractérisée en ce que les ions métalliques divalents sont les ions $Mn^{2+}$ ou $Mg^{2+}$.

9. Utilisation selon la revendication 3, caractérisée en ce que les nucléosides diphosphates isotopiquement marqués sont obtenus par réaction d'échange entre le $\beta$-phosphate d'un nucléoside diphosphate et de l'orthophosphate isotopiquement marqué.

10. Nucléosides diphosphates isotopiquement marqués obtenus par dégradation phosphorolytique de substrats constitués de nucléosides oligophosphates contenant deux nucléosides reliés entre eux par leurs carbones 5' par l'intermédiaire d'une chaîne comprenant au moins 4 résidus phosphates à l'aide de la 5',5'''-$P^1$,$P^4$-diadénosine-tétraphosphate $\alpha,\beta$-phosphorylase.

11. Nucléosides diphosphates isotopiquement marqués obtenus par réaction d'échange entre le $\beta$-phosphate d'un nucléoside diphosphate et de l'orthophosphate isotopiquement marqué à l'aide de la 5',5'''-$P^1$,$P^4$-diadénosine-tétraphosphate $\alpha,\beta$-phosphorylase.

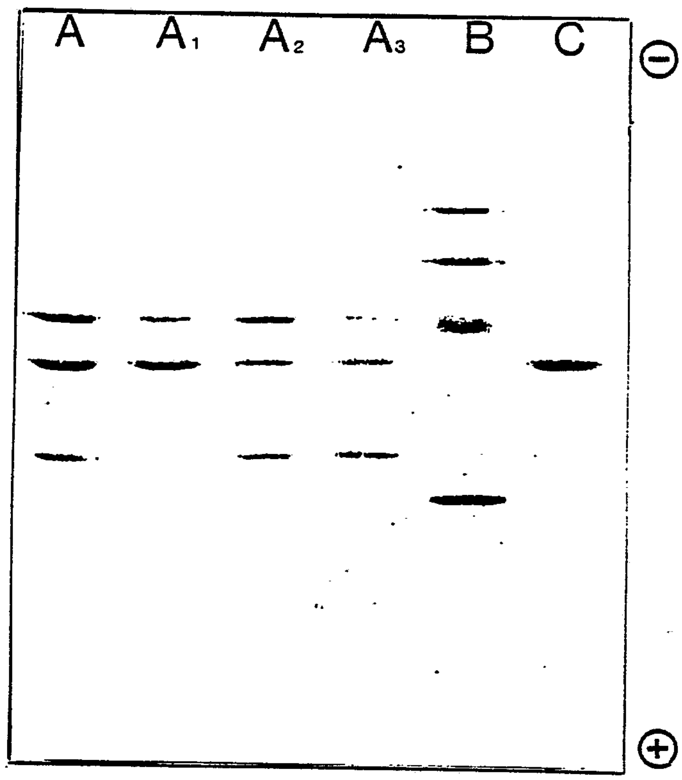


Fig. 1

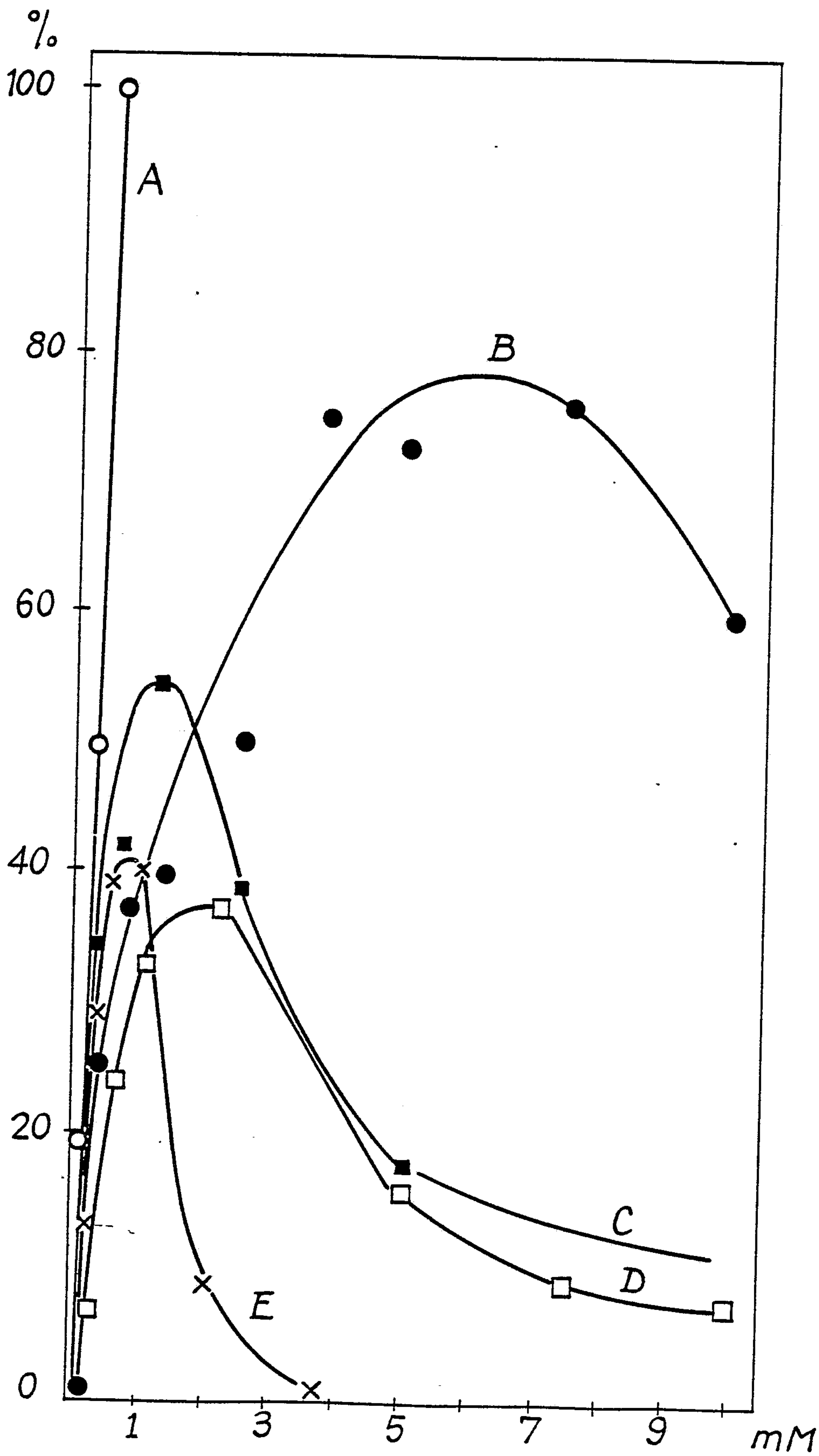


Fig.2

Office européen des brevets

# RAPPORT DE RECHERCHE EUROPEENNE

Numéro de la demande

EP 86 40 0270

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | CHEMICAL ABSTRACTS, vol. 92, no. 19, 12 mai 1980, pages 206,207, no. 159422k, Columbus, Ohio, US; G. SMITH et al.: "A direct rapid radioassay for adenosine", & CLIN. CHIM. ACTA 1980, 101(2-3), 287-91<br>* Résumé *<br>--- | 10,11 | C 12 N 9/12<br>C 12 P 19/30<br>C 07 B 59/00 //<br>(C 12 N 9/12<br>C 12 R 1:865) |
| P,X | THE JOURNAL OF BIOLOGICAL CHEMISTRY, vol. 260, no. 6, 25 mars 1985, pages 3542-3547, The American Society of Biological Chemists Inc., US; A. GURANOWSKI et al.: "Phosphorolytic cleavage of diadenosine 5',5'''-P1,P4-tetraphosphate"<br>* En entier *<br>--- | 1-9 | |
| A | BIOCHEMISTRY, vol. 22, 1983, pages 4388-4394, American Chemical Society, US; A.L. HAAS et al. "Ubiquitin adenylate: Structure and orle in ubiquitin activation"<br>--- | | DOMAINES TECHNIQUES RECHERCHES (Int. Cl.4)<br><br>C 12 N |
| A | EUR. J. BIOCHEM., vol. 136, 1983, pages 469-479, FEBS; J.J. LED et al.: "Phosphorolytic activity of Escherichia coli glycyl-tRNA synthetase towards its cognate aminoacyl adenylate detected by 31P-NMR spectroscopy and thin-layer chromatography"<br>----- | | |

Le présent rapport de recherche a été établi pour toutes les revendications

| Lieu de la recherche | Date d'achèvement de la recherche | Examinateur |
|---|---|---|
| LA HAYE | 26-05-1986 | DESCAMPS J.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82